Europäisches Patentamt

European Patent Office (11) Publication number: **0 299 242**

Office européen des brevets **A2**

# EUROPEAN PATENT APPLICATION

(21) Application number: **88109945.1**

(22) Date of filing: **22.06.88**

(51) Int. Cl.⁴ **C12N 15/00 , A61K 39/385 , A61K 39/29 , A61K 39/015 , A61K 39/21 , A61K 39/13 , A61K 39/135 , A61K 39/295**

(30) Priority: **22.06.87 EP 87108915**
**22.06.87 EP 87108914**

(43) Date of publication of application:
**18.01.89 Bulletin 89/03**

(84) Designated Contracting States:
**AT BE CH DE ES FR GB GR IT LI LU NL SE**

(71) Applicant: **MEDICO LABS AG**
**Freigutstrasse 22**
**CH-8039 Zürich(CH)**

(72) Inventor: **Thoma, Hans A. Dr.**
**Giselastrasse 3/V**
**D-8000 München 40(DE)**

(74) Representative: **Strehl, Schübel-Hopf, Groening, Schulz**
**Maximilianstrasse 54 Postfach 22 14 55**
**D-8000 München 22(DE)**

(54) **Heterologous viral peptide particle immunogens.**

(57) An immunogenic particle comprises a plurality of first peptide monomers and a plurality of second peptide monomers, each of said first peptide monomers comprising :

an amino acid sequence corresponding to a substantial portion of a peptide which upon secretion will form particles which are at least 10 nm in diameter, and

an amino acid sequence corresponding to a heterologous epitope,

each of said second peptide monomers comprising an amino acid sequence corresponding to a substantial portion of a peptide which upon secretion will form particles which are at least 10 nm in diameter, each of said second peptide monomers being free of an amino acid sequence corresponding to a heterologous epitope, wherein said first peptide monomer and said second peptide monomer are not identical but are capable of binding together.

EP 0 299 242 A2

## HETEROLOGOUS VIRAL PEPTIDE PARTICLE IMMUNOGENS

### FIELD OF THE INVENTION

This invention relates to particles which are useful as immunogens. Specifically, the particles contain as monomers peptides which present epitopes from one or more viruses or parasites [e.g., Hepatitis (B, A, non-A, non-B), AIDS (HIV, HTLVIII), malaria, foot and mouth disease, polio] to which an immune response is to be evoked.

### BACKGROUND OF THE INVENTION

Peptide particles similar to the naturally-occurring 20 nm particle isolated from blood of individuals infected with the Hepatitis B virus ("HBV") have been found to be useful as immunogens. For example, Murray, U.S. Patent No. 4,710,463 discloses recombinant methods for producing the S-peptide of HBV which can be used to form particles which are useful as an immunogen. Valenzuela, et al., European Patent Publication 0 175 261, disclose recombinant methods for producing peptides containing epitopes from certain viruses and parasites coupled to the S protein from HBV which form particles which are claimed to be useful as immunogens.

These types of particles are most effective as immunogens when the cells expressing the peptides which will form the particles express the various peptides in proportions which allow incorporation into the resulting particles of a large percentage of peptides containing the desired epitopes. Valenzuela et al. incorporate the entire S peptide in all of the monomers used to make particles. The presence of the strong S initiation region in the Valenzuela et al. constructs can serve as a start signal for transcription which will produce monomers which do not contain the heterologous (not HBV) epitopes contained in the construct, resulting in particles containing a reduced level of monomers having the heterologous epitopes.

### SUMMARY OF THE INVENTION

The term "epitope" as used herein refers to a sequence of at least six consecutive amino acids encoded by the genome or a specifically designated region of the genome, of a designated virus (e.g., "HAV epitope" refers to a sequence of at least six consecutive amino acids encoded by the genome of Hepatitis A virus). The term "heterologous epitope" as used herein refers to an epitope from any source other than Hepatitis B virus. The term "T-cell epitope" as used herein refers to an epitope that interacts with receptors on the surfaces of T-cells to enhance or otherwise effect an immune response.

In accordance with the present invention, immunogenic particles are disclosed comprising a plurality of first peptide monomers and a plurality of second peptide monomers bound together. Each of the first peptide monomers comprises an amino acid sequence corresponding to a substantial portion of a peptide which upon secretion will form particles which are at least 10 nm in diameter, and an amino acid sequence corresponding to a heterologous epitope. Each of the second peptide monomers (1) comprises an amino acid sequence corresponding to a substantial portion of a peptide which upon secretion form particles which are at least 10 nm in diameter, and (2) is free of an amino acid sequence corresponding to a heterologous optiope. The first peptide monomers and second peptide monomers are not identical (i.e., contain different amino acid sequences).

Immunogenic particles are also disclosed comprising a plurality of first peptide monomers, a plurality of second peptide monomers and a plurality of third peptide monomers bound together. The first and second peptide monomers are as described above, with the exception that the first and second peptide monomers can be identical. The third peptide monomers comprise an amino acid sequence corresponding to a substantial portion of a peptide which upon secretion will form particles which are at least 10 nm in diameter, and an amino acid sequence corresponding to a T-cell epitope.

Other immunogenic particles are disclosed comprising a plurality of first peptide monomers and a plurality of second peptide monomers bound together. Each of the first peptide monomers comprises an

2

amino acid sequence corresponding to a substantial portion of a peptide which upon secretion will form particles which are at least 10 nm in diameter, an amino acid sequence corresponding to heterologous epitope, and an amino acid sequence corresponding to a T-cell epitope. Each of the second peptide monomers (1) comprises an amino acid sequence corresponding to a substantial portion of a peptide which upon secretion will form particles which are at least 10 nm in diameter, and (2) is free of an amino acid sequence corresponding to a heterologous epitope.

Peptides which upon secretion will form particles which are at least 10 nm in diameter include HBV S peptide, HBV core antigen, polio surface antigen, Hepatitis A surface antigen, Hepatitis A core antigen, HIV surface antigen and HIV core antigen. Preferably, the peptide forming at least 10 nm particles is HBV S peptide. Immunogenic particles are also disclosed as described above wherein the peptide which upon secretion will form particles which are at least 10 nm in diameter is HBV S peptide. In some such immunogenic particles amino acid sequence is present corresponding to only a substantial portion of the HBV S peptide; in other particles amino acid sequence is present corresponding to all of the HBV S peptide.

The heterologous epitope in immunogenic particles of the present invention can be a viral or parasitic epitope. Viral epitopes include epitopes selected from the group consisting of a Hepatitis A epitope, a non-A Hepatitis epitope, a non-B Hepatitis epitope, an HIV epitope, a polio epitope and a foot and mouth disease virus epitope. A malaria epitope is an example of a parasitic epitope. The nucleotide sequences of preferred heterologous epitopes are listed in Table I. Other known or later-discovered heterologous epitopes can also be employed in practicing the present invention regardless of whether such epitopes are of viral or parasitic origin.

The nucleotide sequence of preferred T-cell epitopes are listed in Table I.

Recombinant DNA molecules are also disclosed comprising a monomer coding region. The monomer coding region comprises a first DNA sequence and a second DNA sequence. The first DNA sequence encodes for expression of an amino acid sequence corresponding to a substantial portion but not all of the HBV S peptide. The second DNA sequence encodes for expression of an amino acid sequence corresponding to a heterologous epitope. The first DNA sequence and second DNA sequence (1) are in the same reading frame, (2) encode for respective discrete regions of a single peptide, and (3) are operatively linked to an expression control sequence in the recombinant DNA molecule. In certain such recombinant DNA molecules the monomer coding region consists essentially of the first DNA sequence and the second DNA sequence.

Recombinant DNA molecules are disclosed comprising a first DNA sequence and a second DNA sequence. The first DNA sequence encodes for expression of an amino acid sequence corresponding to a substantial portion of the HBV S peptide. The second DNA sequence encodes for expression of an amino acid sequence corresponding to a T-cell epitope. The first DNA sequence and encode for respective discrete regions of a single peptide, and (3) are operatively linked to an expression control sequence in the recombinant DNA-molecules.

Other recombinant DNA molecules are disclosed comprising a first DNA sequence, a second DNA sequence and a third DNA sequence. The first DNA sequence encodes for expression of an amino acid sequence corresponding to a substantial portion of the HBV S peptide. The second DNA sequence encodes for expression of an amino acid sequence corresponding to a heterologous epitope. The third DNA sequence encodes for expression of an amino acid sequence corresponding to a T-cell epitope. The first DNA sequence, second DNA sequence and third DNA sequence (1) are in the same reading frame. (2) encode for respective discrete regions of a single peptide, and (3) are operatively linked to an expression control sequence in said recombinant DNA molecule.

Host cells are disclosed which have been transformed with a recombinant DNA molecule described above. Host cells are also disclosed which have been cotransformed with a recombinant DNA molecule as described above and a second recombinant DNA molecule comprising a DNA sequence encoding for expression of an amino acid sequence corresponding to a substantial portion of or all of the amino acid sequence of the HBV S peptide.

Peptides are disclosed comprising a first discrete region and a second discrete region. The first region corresponds to the amino acid sequence of a heterologous epitope and the second region corresponds to a substantial portion but not all of the HBV S peptide. Other peptides are disclosed which consist essentially of the first region and the second region.

Peptides are also disclosed comprising a first discrete region and a second discrete region. The first region corresponds to the amino acid sequence of a T-cell epitope and the second region corresponds to a substantial portion of the HBV S peptide.

Other peptides are disclosed comprising a first discrete region, a second discrete region and a third discrete region. The first region corresponds to the amino acid sequence of a heterologous epitope. the second region corresponds to the amino acid sequence of a T-cell epitope and the third region corresponds to a substantial portion of the HBV S peptide.

Pharmaceutical preparations and preparations useful for production of antibodies are disclosed. The preparations comprise immunogenic particles as described above in sufficient concentration to elicit an immune response upon administration of the preparation and a suitable carrier. Preparations useful for production of antibodies are also disclosed comprising peptides as described above in sufficient concentration to elicit an immune response upon administration of the preparation and a suitable carrier.

Processes for producing transfected cells are disclosed comprising providing host cells which have been made competent for uptake of DNA. exposing the host cells to a first preparation of DNA comprising a recombinant DNA molecule described above, allowing under suitable conditions the host cells to take up DNA from the first preparation of DNA, and selecting for host cells which have taken up exogenous DNA. The first DNA preparation can also further comprise a DNA molecule encoding for a peptide including the amino acid sequence of the HBV S peptide. The processes can also further comprise. either before or after exposing them to the first DNA preparation, exposing the host cells to a second DNA preparation and allowing under suitable conditions the host cells to take up DNA from the second preparation of DNA. The second preparation of DNA comprises a DNA molecule encoding for a peptide including the amino acid sequence of the HBV S peptide.

Methods of producing a peptide are also disclosed comprising preparing a recombinant DNA molecule described above, transfecting a host cell with the recombinant DNA molecule. culturing the host cell under conditions allowing expression and secretion of protein by the host cell, and collecting the peptide produced as a result of expression of DNA sequences within the recombinant DNA molecule. The peptide produced by such method can contain less than the entire amino acid encoded by the coding region of the recombinant DNA molecule. This may result from transcription and/or translation of only a portion of the coding region of the recombinant molecule or by deletions made in the peptide after translation.

A method of producing immunogenic particles is disclosed comprising preparing a recombinant DNA molecule described above, transfecting a host cell with the recombinant DNA molecule. culturing the host cell under conditions allowing expression and secretion of protein by the host cell. and allowing under suitable conditions the aggregation of peptide monomers produced as a result of expression of exogenous DNA sequences within the host cell. The host cells can also be additionally transfected with a DNA molecule encoding for a peptide including he amino acid sequence of the HBV S peptide.

Methods of manufacturing pharmaceutical preparations and preparations useful for producing antibodies are disclosed comprising preparing a recombinant DNA molecule described above. transfecting a host cell with the recombinant DNA molecule, culturing the host cell under conditions allowing expression and secretion of protein by the host cell, allowing under suitable conditions the aggregation of peptides produced as a result of expression of DNA sequences within the host cell to form immunogenic particles. and combining the immunogenic particles with a suitable carrier such that the immunogenic particles are present in sufficient concentration to cause production of antibodies upon administration of the preparation to an individual. The host cells can also be additionally transfected with a DNA molecule encoding for a peptide including the amino acid sequence of the HBV S peptide.


## DESCRIPTION OF THE PREFERRED EMBODIMENTS


Recombinant constructs of the present invention can be prepared using any viral epitope for which the nucleotide sequence can be determined or isolated. Preferred epitopes for practicing the present invention are from HBV, Hepatitis A virus ("HAV"), the AIDS virus ("HIV"), malaria, poliovirus and the foot and mouth disease virus. Preferred epitopes (both T-cell and B-cell epitopes) and their sequences are summarized in Table IA.

## Table IA (continued)

| Oligo. No. | Source | Amino Acid Nos. | Sequence (5'-3') |
|---|---|---|---|
| 139 | HIV | 596-631 | GATCTAACATGGAGAACGTCGACCAAC AGCTCCTGGGGATTTGGGGGTTGCTCTGGAA AACTCATTTGCACCACTGCTGTGCCTTGG AATGCTAGTTGGAGTAATAAATCTCTGG AACAGATTTGGAA(T) |
| 141 | HIV | 616-646 | GATCTAACATGGAGAACXXXXXXCCTTG GAATGCTAGTTGGAGTAATAAATCTCTG GAACAGATTTGGAATAACATGACCTGG ATGGAGTGGGACAGAGAAATTAACAAT TACAC(A) |
| 143 | HIV | 646-674 | GATCTAACATGGAGAACACAAGCTTAA TACACTCCTTAATTGAAGAATCGCAAA ACCAGCAAGAAAAGAATGAACAAGAA TTATTGGAATTAGATAAATGGGC(C) |
| 145 | HIV | 666-701 | GATCTAACATGGAGAACXXXXXXGAATT ATTGGAATTAGATAAATGGGCAAGTTTG AATTGGTTTAACATAACAAATTGGCTGT GGTATATAAAATTATTCATAATGATAG TAGGAGGCTTGGATGG(T) |
| 147 | HIV | 695-734 | GATCTAACATGGAGAACXXXXXXATAGT AGGAGGCTTGGTAGGTTTAAGAATAGTT TTTGCTGTACTTTCTGTAGTGAATAGAGT TAGGCAGGGATATTCACCATTATCGTTTC AGACCCACCTCCCAATCCCGAGGGGACC (C) |
| 149 | HIV | 729-753 | GATCTAACATGGAGAACXXXXXXCCAAT CCCGAGGGGACCCGACAGGCCCGAAGGA ATAGAAGAAGGTGGAGAGAGAGACAGA GACAGATCCAT(T) |
| 155 | malaria | | GATCTTTAACATGGAGAACXXXXXXAAC GCTAACCCTAACGCTAACCCTAACGCTA ACCCTAACGCTAACCCTAACGCTAACCC TAACGCTAACCCTAACGCTACCCCT(X) |
| 161 | foot/mouth | 141-160 | GATCTTAACATGGAGAACXXXXXX- nucleotides coding for amino acids 141-160-X |
| 167 | poliovirus | | GATCTTAACATGGAGACCGCCGCCGCCG GATCCGATAACCCAGCGTCGACCACGAA TAAGGATAAGGGATCCGCCGCCGCCGCCG CCGCCGCCGCCGCCGCC(T) |
| 175 | HBV pre-S1 (T-cell) | | GATCTTTAACATGGAGAACAATCCTCTG GGATTCTTTCCCGATCACCAGTTGGATCC AGCCTTCAGAGCAAACACCGCAAATCC AGATTGGGACTTCAATCCCAG(T) |

## Table IA

| Oligo. No. | Source | Amino Acid Nos. | Sequence (5'-3') |
|---|---|---|---|
| 105 | HAV | 500-532 | GATCTAACATGGAGAACTCAACACAG TTTCTACAGAGCAGAATGTTCCAGATC CCCAAGTTGGTATAACAACCATGAAGG ATTTAAAAGGCAAAGCTAATAGGGGA AAAATGGA(T) |
| 107 | HAV | 511-539 | GATCTTTAACATGGAGACGAATTCCGA TCCCCAAGTTGGTATAACAACCATAAA GGATTTAAAAGGCAAAGCTAATAGGGG AAAAATAGATGTTTCAGGAGTGCAACC CTCGAG(T) |
| 109 | HAV | 529-560 | GATCTAACATGGAGAACXXXXXXGGAAA AATGGATGTTTCAGGAGTGCAAGCACCT GTGGGAGCTATCACAACAATTGAGGATC CTGTTTTAGCAAAGAAAGTACCTGAGAC ATTCCC(C) |
| 111 | HAV | 555-585 | GATCTAACATGGAGAACXXXXXXGTACC TGAGACATTCCCTGAATTGAAACCTGGA GAGTCCAGGCATACATCAGATCATATGT CTATTTACAAGTTTATGGGAAGGTCTCA TTT(T) |
| 113 | HAV | 580-615 | GATCTAACATGGAGAACXXXXXXATGGG AAGGTCTCATTTTTTGTGCACTTTTACTT TTAATTCAAACAATAAAGAGTACACA TTTCCTATAACCTTGTCTTCAACCTCCAA TCCTCCTCATGGTTTACC(C) |
| 131 | HIV | 500-524 | GATCTAACATGGAGAACXXXXXXCCATT AGGAGTAGCACCCACCAAGGCAAAGAG AAGAGTGGTCCAGAGAGAAAAAAGAGC AGTGGGAATAGGAGC(T) |
| 133 | HIV | 514-553 | GATCTAACATGGAGAACXXXXXXAGAGA AAAAAGAGCAGTGGGAATAGGAGCTTT GTTCTTCCTTGGGTTCTTGGGAGCAGCAGG AAGCACTATGGGGGCAGCGTCAATGACG CTGACGGTACAGGCCAGACAATTATTGT C(T) |
| 135 | HIV | 539-577 | GATCTAACATGGAGAACXXXXXXGCAGC GTCAATGACGCTGACGGTACAGGCCAGA CAATTATTGTCTGGTATAGTGCAGCAGC AGAACAATTTGCTGAGGGCTATTGAGGC GCAACAGCATCTGTTGCAACTCACAGT (C) |
| 137 | HIV | 574-602 | GATCTAACATGGAGAACXXXXXXCAACT CACAGTCTGGGGCATCAAGCAGCTCCAGG CAACAATCCTGGCTGTGGAAAGATACCT AAAGGATCAACAGCTCCTGGGGAT(T) |

## Table IA(continued)

| Oligo. No. | Source | Amino Acid Nos. | Sequence (5'-3') |
|---|---|---|---|
| 177 | HBV pre-S2 (T-cell) | | GATCTTTAACATGGAGAACCAGTGGAAT TCCACAACCTTCCACCAAACTCTGCAAG ATCCCAGAGTGAGAGGCCTGTATTTCCCT GCTGGTGGCTCCAG(T) |
| 179 | malaria (T-cell) | 326-343 | GATCTTAACATGGAGAACXXXXXXX- nucleotide sequence coding for amino acids 326-343-XXXX |
| 181 | malaria (T-cell) | 103-116 | GATCTTAACATGGAGAACXXXXXXX- nucleotide sequence coding for amino acids 103-116-GCCGCCGCCGCCGCCGCC GCCGCCGCCGCCX |
| 185 | HBV core | | GATCTTAACATGGAGAACXXXXXXX- nucleotide sequence of HBV core protein BglII/ApaI 620bp fragment- XXX |
| 189 | malaria (T-cell)/ HBV pre-S1 (T-cell) | | GATCTTAACATGGAGAACXXXXXXX- GGATTCTTTCCCGATCACCAGTTGGATCC AGCCTTCAGAGCAAACACCGCAAATCC AGATTGGGACTTCAATCCCAG(T) GCCGCCGCCGCCGCCGCCGCCGCC GATCTTAACATGGAGAACXXXXXXX- nucleotide sequence coding for amino acids 326-343-XXXX |
| 201 | HBV pre-S2 (T-cell)/ HIV | | GATCTAACATGGAGAACXXXXXXXCCATT AGGAGTAGCACCCACCAAGGCAAAGA GAAGAGTGGTCCAGAGAGAAAAAAGAG CAGTGGGAATAGGAGAATCCTCTGGGAT TCTTTCCCGATCACCAGTTGGATCCAGCC TTCAGAGCAAACACCGCAAATCCAGAT TGGGACTTCAATCCCAG(T) |

The epitopes listed in Table IA may be used in designing constructs of the present invention as specifically described below or in other combinations. Adapter or linker sequences (e.g., restriction fragments or synthetic obigonucleotides, other than those described below) can also be used to insert the epitopes listed in Table IA or other desired epitopes into constructs of the present invention. All examples can be performed with the MT-promoter and other promoters as well.

## Example 1

Vectors used for the expression of a peptide wherein the heterologous epitope is of HAV origin

### Plasmid pHB1

The H2K promoter was isolated as an EcoRI.BgIII fragment (-kb) from psp 65 H2 (available from Exogen). The promoter ligated with a 2.3 kb BgIII.BgIII fragment from the HBV genome and a fragment from pBR322 including the selection marker and the origin of replication.

### Plasmid pHA1

A pBR322 fragment with sticky ends after restriction digestion with the endonucleases EcoRI and BamHI was ligated with a synthetic adapter sequence:

EcoRI                                                         BglII

```
5' AATTCAAGATCTGCCATGCAGTGGAAGCTTAAGGATCCTCTAGA 3'
       GTTCTAGACGGTACGTCACCTTCGAATTCCTAGGAGATCTCTAG
```

and a HindIII-BamMI fragment (310 bp) from the HAV genome. The adapter guarantees that the HindIII.BamHI fragment of HAV is in the exact reading frame of the start signal whereby the starting signal and the flanking sequences of it are from the HBV surface antigen ("HBV-S-Ag, HBs or HBsAg") (subtype ayw). The new plasmid was digested with BgIII and XbaI (322 bp) and the 333 bp fragment was isolated after agarose gel electrophoresis containing the HAV-gene coding region.

### Plasmid pHBA2

This vector contains the HBK promotor sequence which is cloned into a pBR322 part between the EcoRI and BgIII sites, and a gene sequence coding for example for the HBV-S peptide (2.3 kb BgIII fragment, isolated from the HBV-genome, subtype ayw). A part of the 5′ terminal sequence of the HBV fragment was deleted (550 bp) by partial digestion with BgIII and XbaI and replaced by the DNA fragment (322 bp) isolated from plasmid pHAI coding for part of the VP1 peptide of the HAV with a start signal upstream of the HAV gene. The replacement was set in the same reading frame as was the deleted fragment.

### Plasmids pHBA3, pHBA4, pHBA5, pHBA6, pHBA7

Plasmid pHBA3, pHBA4, pHBA5, pHBA6 and pHBA7 were constructed as was pHBA2 with substitution of oligonucleotide Nos. 105, 107, 109, 11 and 113, respectively, from Table I in place of the 322bpHAVfragment.

### Plasmids pHBA2S1, pHBA3S1, pHBA4S1, pHBA5S1, pHBA6S1, pHBA7S1

Plasmids pHBA2, pHBA3, pHBA4, pHBA5, pHBA6 and pHBA7 were each linearized with EcoRI. Each of these EcoRI fragments was ligated together with a second EcoRI fragment isolated from plasmid pMT-neo-K (available from ATCC) containing the MT-promoter upstream of the neomycin selection gene.

### Plasmids pHBA2A and pHBA3A

Plasmids pHBA2 and pHBA3A were linearized with EcoRI. Each of these fragments was ligated together with a second EcoRI fragment isolated from a plasmid pdhfr 3.2 (available from ATCC) containing the dhfr selection (amplification) gene.

Plasmids pHBA2S2 and pHBA3S2

Plasmids pHBA2 and pHBA3 were linearized with EcoRI. Each of these fragments was ligated together with a second EcoRI fragment isolated from a plasmid pMTegpt (available from Pharmacia) containing the MT-promoter upstream of the egpt selection gene.

## Example 2

Vectors used for the expression of a peptide containing a T-cell epitope

1) T-cell epitope is of HBV-pre-S₁ origin

Plasmid pUT2B

The plasmid pMT-neoK was digested with EcoRI and BglII. Two molecules were expected, one (1.9 kb) carrying the MT-promoter was removed from the fragment of interest which carries a pBR part and the neomycin selection gene by agarose gel electrophoresis. This fragment was ligated together with another fragment (330 bp) carrying the U2-promoter sequence. The U2 promoter was isolated from plasmid pUC-8-42 (available from Exogen) as a EcoRI-ApaI fragment. The new plasmid was called pU2-neo.

In a second step this plasmid pU2-neo was digested with BglII and BamHI to remove the neomycin gene from the plasmid and the fragment of interest containing the pBRS22 part and the U2 promoter was ligated together with the 2.3 kb BglII-HBV-fragment (as described above). This plasmid which is called pUB was digested with BglII and XbaI to delete the 550 bp (as described above). The deletion was replaced ba a synthetic oligo DNA sequence coding for the T cell epitope of HBV pre-S₁ including the start signal and flanking sequences of the natural pre-S₁ and with corresponding sticky ends. The new plasmid was called PUT2B1 (oligonucleotide No. 175 from Table IA).

2) wherein the T-cell epitope is of malaria origin, HBV-pre-S2 origin, or HBV-core origin.

Plasmids pUT1B, pUT3B. pUT4B

Plasmids pUT1B, pUT3B and pUT4b were prepared in the same way as described above, for pUT2B1. Only the replacing synthetic oligo-DNA sequences differ in that the DNA sequences are coding for different T- cell epitopes (for malaria: oligo no. 179; for HBV-pre-S2: oligo no. 177; for HBV-core; oligo no. 185; all from Table I A).

## Example 3

Vectors used for the expression of a particle forming polypeptide of HBV origin

9

Plasmid pHBP1

The plasmid pHB1 was digested with BgIII and XhoI. Two molecules were expected, one of 430 bp (containing the pre-S sequence), was removed and separated from the fragment of interest by agarose gel electrophoresis. The larger fragment of interest was ligated to a synthetic adapter sequence:

$$5' \text{ GATCTTTAAC } 3'$$
$$\text{AAATTGAGCT}$$
$$\text{BgIII} \qquad \text{XhoI}$$

to obtain a closed circle of plasmid DNA.


Plasmid pMBP1

The plasmid pHBP1 was digested with EcoRI and BgIII. Two molecules were generated, one containing the H2K-promoter (2 kb) was removed and replaced by an EcoRI-BgIII fragment (1.9 kb) which contains the MT-promoter which was isolated from the plasmid pMT-neo.


Plasmid pMBP2

The plasmid pMT-neo was digested with BamHI and BgIII and two molecules were generated. One containing the neomycin selection gene was removed and the fragment of interest (4.5 kb) containing the MTpromoter and a pBR part was isolated after agarose gel electrophoresis and ligated together with a fragment coding for the HBV-S-Ag (subtype adw). The HBV-S-Ag fragment (1.8 kb) was isolated from the HBV genome by digestion with the endonucleases DgIII and BamHI.


Plasmid pHBP2

The plasmid pH-neo (available from ATCC) was digested with BgIII and BamHI. Two molecules were expected, one containing the neomycin selection gene was removed and the fragment of interest, containing the H2-promoter and a pBR part was isolate. Said fragment was ligated together with a fragment isolated from HBV genome (adw subtype) of 1.8 kb coding for the HBV-S-Ag (as described above).


Plasmid pHBC1

The plasmid pH-neo was digested with BgIII and BamHI. Two molecules were expected, one containing the neomycin selection gene was removed and the fragment of interest, containing the H2-promoter and a pBR part was isolated. Said fragment was ligated together with a BamHI fragment (1.776 Kb) isolated from HBV genome (subtype adw), coding for the HBV-core peptide.


Plasmid pHBP1S1

The plasmid pHBP1 was linearized with EcoRI and ligated together with a second EcoRI fragment isolated from the plasmid pMT-neo-K containing the MT-promoter upstream of the neomycin selection gene.


Plasmids pMBP1S1, pHBP2S1, pMBP2S1, pHBC1S1

Plasmids pMBP1, pHBP2, pMBP2 and pHBC1 were supplemented by adding the gene sequence coding for the neomycin selection gene as described for the plasmid pHBP1S1.

Plasmid pHBP1S2

The plasmid pHBP1 was linerized with Eco RI and ligated together with a second EcoRI fragment isolated from the plasmid pMT-egpt-K (available from Pharmacia) containing the MT-promoter upstream of the egpt selection gene.

Plasmids pMBP1S2, pHBP2S2 and pMBP2S2

Plasmids pMBP1, pHBP2 and pMBP2 were supplemented by adding the gene sequence coding for the egpt selection gene as described for the plasmid pHBP1S2.

Plasmid pHBP1A

The plasmid pHBP1 was linearized with EcoRI and ligated together with a second EcoRI fragment isolated from the plasmid pdhfr 3.2 containing the selection/amplification gene dhfr.

Plasmids pMBP1A, pHBP2A, pMBP2A

Plasmids pMBP1, pHBP2 and PMBP2 were supplemented by adding the gene sequence coding for the selection/amplification gene dhfr as described for the plasmid pHBP1A.

<u>Example 4</u>

Generation of antigen producing recipient cells by gene transfer (transfection) of desired plasmids into host cells (heterologous HAV epitope and T-cell epitope)

(1) The recipient cells (C127, L, CHO, (ATCC)) were first transfected (as described below) with the plasmid pHBP2S2 (containing the gene sequence of the particle forming polypeptide). A cell clone was found which expressed on a high level said particles.
A second transfection (co-transfection) of the identified clone was done with the plasmids pHBA2A1 and pUT2B. The co-transfected cell clones were analysed by immunoassays to detect cells which were secreting various monomers encoded by the co-transfected plasmids.
(2) The recipient cells were directly transfected with a mixture or the three desired plasmids (pHBP2S1, pHBA2 and pUT2B). After transfection, the grown cell clones were directly analysed by immunoassays to detect cell clones secreting the various monomers encoded by the co-transfected plasmids.
(3) The same collection of monomers will be obtained after transfection of the recipient cells when the following plasmid combinations were used for the transfection:
a) first pMBP2S1, then pHBA2S2 and pUT2B
b) mixture of pHBP2A, pHBA2 and pUT2B
Plasmids pHBA3, pHBA4 or pHBA5 can also be substituted for pHBA2 in the above-described transfection schemes.
(4) A similar collection of monomers will be also obtained after two transfections of the recipient cells with the following plasmid combinations:
-first transfection with: pHBP2S2 + pUT2B
-second transfection with: pHBA3S1 + pUT1B
Transfections were performed as follows. The recipient cells were seeded in normal growth medium (DMEM + 5% Fetal Calf Serum, Glycose and Glutamin) into petri-dishes (1-2 x 10$^6$ cells per 10 cm dish) at day 1.
The next day the medium was removed (4 hours before the DNA precipitate was added onto the cells), and the cells were washed twice with 1 x PBS. Then 8 ml DMEM (complemented with Hepes final concentration 10 nM) without FCS was added. 4 hours later the DNA precipitate was added to the cells. Again after 4 hours the medium was removed and 3 ml of Glycerol-Mix (50 ml 2 x HBS buffer. 30 ml glycerol. 120 ml distilled water) was added. The Glycerol-Mix was immediately removed after an incubation at 37 C for 3 minutes and the cells were washed with 1 x PBS. The cells were cultivated overnight with 8 ml of DMEM with 10% FCS. The following day the cells were recovered from the dish by treating with Trypsin-EDTA-Solution (0.025% Tryosin + 1 mM EDTA). Afterwards, to remove the Trypsin-EDTA, the cells were washed with 1 x PBS, suspended in DMEM with 10% FCS and distributed into 24-well-plates (cells from one dish into one 24-well-plate). When the cells had grown well, selection medium was added (concentration 0.5 - 1 mg/ml of neomycin and Xanthine: 250mg/ml, Hypoxanthine:15mg/ml, (or adenenine:25mg/ml), Thymindine:10mg/ml, Aminopterine:2mg/ml, Mycophenolicacid:25mg/ml, for esample).

The medium was changed every week. The first growing cell colonies were seen after 2 weeks. The grown cell clones were screened for a protein secretion by ELISA.

DNA precipitate was prepared for transfection as follows. To 10 ug of plasmid DNA and 20 ug of Carrier-DNA (salmon-sperm DNA, calf-thymus DNA) TE-buffer (10 mM Tris-HC1, 1 mM EDTA, pH 7.05) was added to a final volume of 440 ul and mixed together with 60 ul 2 M CaCl$_2$. Then the same amount of 2 x HBS (Hepes 50 mM. NaCl 280 mM. Na$_2$HPO$_4$ 1.5 mM, pH 7.05) was added and mixed well.

The precipitation solution was incubated for 30 minutes at 37° C and added directly to the cells which were transfected.

Table I summarizes ELISA analysis of particles prepared in accordance with present invention including an HAV heterologous epitope, an HBV-preS, T-cell epitope and HBSAg epitope.

Table I

| Measurement of the desired epitopes after CsCl gradient according to claim: | | | |
|---|---|---|---|
| Measurement by ELISA with Antibodies | | | |
| CsClFraction -No. pooled | anti-HBsAg (OD) | anti-HAV (OD) | anti-HBV-preS1 (OD) |
| 10 -12 | 1.646 | 0.871 | 0.725 |

Example 5

Quantitative Determination of HBsAg

a. with radioimmunoassay

In the AUSRIA II-25 "sandwich" radioimmunoassay (available from ABBOTT), beads coated with guinea pig Antibody to Hepatitis B Surface Antigen (Anti-HBs) were incubated with serum, plasma or purified protein and appropriate controls. Any HBsAG present was bound to the solid phase antibody. After aspiration of the unbound material and washing of the bead, human [125]-I-AntiHBs was allowed to react with the antibody-antigen complex on the bead. The beads are then washed to remove unbound [125]-I-Anti-HBs.

)-Anti-HBs      HBsAg

)-Anti-HBs . HBsAg $^{125}$I-Anti-HBs

)-Anti-HBs . HBsAg . $^{125}$I-Anti-HBs

The radioactivity remaining on the beads was counted in a gamma scintillation counter.

b. with ELISA

In the Enzygnost HBsAG micro "sandwich" assay (available from Behring ). wells were coated with anti-HBs antibodies. Serum, plasma or purified protein and appropriate controls were added to the wells and incubated. After washing, peroxidase-conjugated anti-HBs were reacted with the remaining antigenic determinants. The unbound enzyme-linked antibodies were removed by washing and the enzyme activity on the solid phase was determined. The enzymatically catalyzed reaction of hydrogen peroxide and chromogen was stopped by adding diluted sulfuric acid. The colour intensity was proportional to the HBsAg concentration of the sample and was measured by photometric comparison of the colour intensity of the unknown samples with the colour intensities of the accompanying negative and positive control sera.

## Example 6

Qualitative Determination of HBV-pre-S-Epitopes

a. with anti-HBsAg antibody coated Elisa plates

In the Enzygnost HBsAg-micro "sandwich" assay, wells were coated with anti-HBs. Serum, plasma or purified protein and appropriate controls were added to the wells and incubated. After washing, antibody anti-HAV (rabbit, human) or anti HBV-pre-S (was added and incubated again. After the next washings, peroxidase-conjugated antibodies (goat anti-rabbit, goat anti-human, or goat anti-mouse) were used as an instrument for tho dotootion of the bound anti-HAV and/or anti-HBVpre-S antibodies and therefore for the presence of the heterologous epitopes (HAV) and/or the T-cell epitopes (HBV-pre-S). The unbound enzyme-linked antibodies are removed by washing and the enzyme activity was measured as described above.

b. with anti-pre-S₁-HBsAg coated ELISA plates

In this "sandwich" assay, wells were coated with antibodies anti-pre-S₁-HBs. Purified protein or cell supernatant and appropriate controls were added to the wells and incubated. The unbound protein was removed by washing (2 times) and an anti-HAV antibody (developed in rabbit) was added to the wells. After an incubation, the bound antibodies were detected with an anti-rabbit-antibody antibody which was enzyme conjugated. Then the enzyme activity was measured and calculated as described above.

## Example 7

To characterize the organization within the host cell genome of the vectors of this invention, chromosomal DNA from cell lines producing particles of this invention was isolated and digested with the appropriate restriction enzyme(s) and analysed by the method of Southern (J. Mol. Biol., Vol. 98, pp. 503-517, 1975) which is incorporated herein by reference using a $^{32}$P-labeled DNA probe. Following digestion of the chromosomal DNA (20 ug) with the restriction enzyme BglII, the resulting fragments were separated by 0.7% agarose gel electrophoresis. Thereafter, the DNA is denatured by exposing to 366 nm UV light for 10 minutes and by incubation in a solution of 0.5 M NaOH and 1 M NaCl for 45 minutes. The gels were neutralized by incubation in 0.5 M Tris, 1.5 M NaCl, pH 7.5 for 60 minutes. The DNA was transferred to a nitrocellulose filter by soaking in 3 M NaCl, 0.3 M sodium citrate (20 x SSC) for 20 hours through the gel by covering the top of the nitrocellulose filter with a staple of dry paper towels. The nitrocellulose filter was kept for 2 hours in a vacuum oven at 80 C.
The radioactive DNA probe from the BglII fragment of the pHBV (2.3 kb) was prepared by nick translation.
For hybridization with the DNA probe, the nitrocellulose filter was sealed in a plastic bag containing 10 ml of prehybridization mixture: 50% formamide, 5 x SSC, 50 mM Sodiumphosphate, pH 7.0, 5 x Denhardt's solution, 250 ug ml denatured salmon sperm DNA. The filter was incubated in this mixture for 4 hours at 45°C, after which the prehybridization mixture is replaced by the hybridization mixture: 50% formamide, 5 x SSC, 20 mM Sodiumphosphate, pH 7.0, 1 x Denhardt's solution, 100 ug ml denatured salmon sperm DNA, 5 x 10⁵ cpm ml ³²P-probe. The filter, after incubating in the hybridization mix for 18 hours at 45°C, was washed three times, 5 minutes each, in 0.1 x SSC, 0.1% SDS at 50 C. The filter was dried at 60°C for 10 minutes and exposed to two X-ray films (XAR-5, KODAK) between two intensifying screens and kept at -80°C. The first X-ray film was developed after 3 days' exposure; the second film after 7 days' exposure.

## Example 8

Vaccine Purification Procedures

### a. Fractionated precipitation with polyethylene glycol (PEG)

The supernatant of monomer producing cultures was collected and split into portions of 2,400 ml. To each portion 144 g of PEG 6000 (Serva) were added and dissolved by stirring at room temperature for 20 minutes and was stirred for another 6 hours at 4°C. The precipitate was separated by centrifugation in 500 ml bottles in a GS 3 rotor at 9,000 rpm (15,000 x g) for 30 minutes at 10°C. The supernatant was collected and 144 g of PEG 6000 were added and dissolved as described above. The solution was stirred at 4 C for 3 hours. The precipitate from this solution was harvested as described above except that centrifugation had been done for 60 minutes. The pellet was resuspended in 20 ml of PBS and submitted to gel chromatography.

### b. Gel Chromatography

The material obtained after PEG precipitation was redissolved in PBS and submitted to gel chromatography on A-5m (BioRAd). Column dimensions were 25 x 1000 mm and 480 ml bed volume. In a typical fractionation run 1,000 g of PEG precipitated monomer in 10 to 15 ml was loaded eluted with PBS at a speed of 6 drops min (18 ml h). 3 ml fractions were collected and submitted to a CsCl gradient.

### c. Sedimentation in CsCl Gradient

About 30 fractions covering the peak in column chromatography on A-5m containing prepurified monomer were collected to approximately 100 ml. This solution was adjusted to a density of 1.30 g cc with CsCl and subsequently transferred to a nitrocellulose tube fitting into a SW 27 28 rotor (Beckman). A gradient was set by underlaying 4 ml of a CsCl solution of 1.35 g/cc and by overlaying 4 ml of 1.25 g cc and 4 ml of 1.20 g/cc density. The gradient was run at 28,000 rpm for 50 hours at 10 C. Thereafter the gradient was fractionated and purified monomer floating in the 1.20 g/cc density layer was collected. The solution was desalted by 3 cycles of dialysis in bags against water.

## Example 9

Analytical Procedures

a) Polyacrylamide gel electrophoresis (PAGE)

For analysis by PAGE, aliquots of purified monomer were adjusted to 100 ul/ml. From each sample 10 .j were taken and mixed with 10 ul 5 M DTT in 10% SDS and 10 ul sample buffer. This mixture was boiled for 10 minutes just before loading in order to break the particles and to generate monomeric molecules. The protein was run in 12% polyacrylamide-N.N'-methylenbisacrylamide gel (LKB-Midget-systems) in a buffer system according to Laemmli, which is incorporated herein by reference, at 40 mA and 100 mA and 100 V over 2 to 30 hours.

b) Western Blot Analysis

After gel electrophoresis the unstained SDS-PAGE gel was placed into transfer buffer (25 mM Tris-HCl, pH 8, 192 mm Glycine in 15% methanol) for 15 to 20 minutes at room temperature. The transfer membrane (Millipore, Immobilon PVDF) was cut into a piece which sized the gel. Then the membrane was pre-wet in 100% methanol and immediately placed into distilled water for rinsing followed by an equilibration for 10 to 15 minutes like the gel. The blotting cassette was assembled, transfer buffer was added and the protein transfer was done by 25 V and 200 to 700 mA for 2 hours. The wet membrane was cut into 3 to 4 identical pieces which were rinsed in water and then placed into protein "blocking" solution (5% BSA in TBS-0.9% NaCl in 20 mM Tris/HCl, pH 7.4) for 1 hour at 37 C with gently agitation. The membrane was wasted 3 times and then incubated with appropriate monoclonal (or polyclonal) antibodies (mouse) for 2 hours at room temperature with gentle agitation. After washing the membrane 3 times again it was incubated with a horseradish-peroxidase conjugated anti-mouse-antibody as described above. After the above step the blots were washed again and incubated with the substrate to develop the colored reaction product. The development of the color took 10 to 20 minutes at room temperature.

## Example 10

Preparation of the Adjuvant of the heterologous polymeric vaccine

To the desired concentration of antigen particles suspended in sterile saline, 1 : 10,000 volume Thimerosol, 1 10 volume of filter-sterilized 0.2 M Al K$(SO_4)_2$ : 12 H20 are added. The pH was adjusted to 5.0 with sterile 1 N NaOH and the suspension is stirred at room temperature for 3 hours. The alum-precipitated antigen was recovered by centrifugation for 10 minutes at 2,000 rpm, resuspended in sterile normal saline containing 1:10,000 Thimerosol and aliquoted under sterile conditions.

## Example 11

Immune Response in Animals after immunisation with the heterologous polymeric vaccine described in Example 4(1) or 4(2).

a. Detection of the anti-HBs response

Male Balb/c mice were immunised i.p. with 0.5 ml suspension containing the desired concentrated of antigen. After 28 days, the mice were bled and the plasma sera were separated by centrifugation for 30 minutes at 6,000 rpm. The antibody content of the sera was measured by using an anti-HBs "sandwich" assay. The wells were coated with HBsAg (subtype adw and ayw). Serum plasma and appropriate controls were added to the wells and incubated. After washing, peroxidase conjugated HBsAg was added which

15

bound to the second determinant of the mouse antibody. The unbound enzyme-linked antigen was removed by washing and the enzyme activity on the solid phase was determined. The enzymatically catalyzed reaction of hydrogen peroxidase and chromogen was stopped by adding diluted sulfuric acid. The colour intensity was proportional to the anti-HBs antibody concentration of the samples and was measured by photometric comparison of the colour intensity of the unknown samples with the colour intensity of the accompanying negative and positive control sera.

b. Detection of the anti-HAV-epitope response

In the peptide "sandwich" assay, wells were coated with peptide which represent the desired heterologous epitope (HAV) and then saturated with a buffer solution containing bovine serum albumin. Serum plasma of the animals and appropriate controls were incubated and binding was detected by a second incubation with an anti-mouseIgG antibody which was enzyme-linked. The unbounded antibodies were removed by washing and the enzyme activity was measured as described above.

c. Detection of the anti-HE-pre-S$_1$ T-cell epitope response

In the peptide "sandwich" assay, wells were coated with peptide which represents the desired T-cell epitope (pre-S1) and then saturated with a buffer solution containing bovine serum albumin. Serum plasma of the animals and appropriate controls were incubated and the binding was detected by a second incubation with an anti-mouse-IgG antibody which was enzyme-linked. The unbounded antibodies were removed by washing and the enzyme activity was measured as described above.

Table II summarizes the immune response of the immunized mice.

Table II

| Immune response in animals - measured antibody titer: | | | |
|---|---|---|---|
| No. of imm. mice | anti-HBsAg (Units/ml) | anti-HBV-pre-S1 (Units/ml) | anti-HAV (Units/ml) |
| 20 | 1.512 | 1.022 | 875 |

Example 12

Immune Response in Animals after Immunisation with the heterologous polymeric vaccine described in Example 4(4).

a. Detection of the anti-HBs response, the anti-HAV-epitope response and the anti-HBV-pre-S$_1$ T-cell epitope response were performed as described above.

b. Detection of the anti-Malaria T-cell-epitope response.

In the peptide "sandwich" assay, wells were coated with peptide which represents the desired T-cell epitope (malaria) and then saturated with a buffer solution containing bovine serum albumin. Serum plasma of the animals and appropriate controls were incubated and the binding was detected by a second incubation with an anti-mouse-IgG antibody which was enzyme-linked. The unbounded antibodies were removed by washings and the enzyme activity was measured as described above.

Table III summarizes the immune response of the immunized mice.

Table III

| Immune Response in Animals - Measured Antibody-Titer | | | | |
|---|---|---|---|---|
| No of mice | anti-HBsAg (Units/ml) | anti-HB-pre-S1 (Units/ml) | anti-HAV (U./ml) | anti-Mal. (U./ml) |
| 20 | 2.317 | 1.536 | 1.274 | 1.046 |

Example 13

Generation of antigen producing recipient cells by gene transfer (transfection) of desired plasmids into host cells (heterologous HAV epitope only) .

To obtain said composed heterologous polymeric vaccine the recipient cells were transfected (cotransfection) with the plasmids pHBA2S1 and pHBP2. The new generated cell clones were analysed by immunoassays to detect cells which were secreting the desired monomers.

The same collection of monomers will be obtained when the following plasmid combinations are used for the transfection of tho recipient cells:

-pHBP2S1 + pHBA2
-pHBP2A + pHBA2
-pHBP2 + pHBA2S1
-pHBP2 + pHBA2S2
-pHBP2 + pHBA2S1

Plasmids pHBA3, pHBA4, pHBA5, pHBA6 or pHBA7 can also be substitued for pHBA2 above.

Table IV summarizes the immune responses of mice immunized with the above described vaccine.

Table IV

| Measurement of desired epitopes after CsCl gradient | | | |
|---|---|---|---|
| Measured by Elisa with Antibodies | | | |
| Fract.-No. pooled | anti-HBsAg (OD) | anti-HAV (OD) | anti-HBV-pre-S1 (OD) |
| 10 - 13 | 1,549 | 0.782 | 0.000 |

Example 14

Immune response in animals after immunisation with the heterologous polymeric vaccine described in Example 13

a. Detection of the anti-HBs response

Male Balb/c mice were immunised i.p. with 0.5 ml suspension containing the desired concentration of antigen. After 28 days, the mice were bled and the plasma sera were separated by centrifugation for 30 minutes at 6,000 rpm. The antibody content of the sera was measured by using an anti-HBs "sandwich" assay. The wells were coated with HBsAg (subtype adw and ayw). Serum plasma and appropriate controls were added to the wells and incubated. After washing, peroxidase conjugated HBsAg was added which bound to the second determinant of the mouse antibody. The unbound enzyme-linked antigen was removed by washing and the enzyme activity on the solid phase was determined. The enzymatically catalyzed reaction of hydrogen peroxidase and chromogen was stopped by adding diluted sulfuric acid. The colour intensity was proportional to the anti-HBs antibody concentration of the samples and were measured by photometric comparison of the colour intensity of the unknown samples with the colour intensity of the accompanying negative and positive control sera.

b. Detection of the anti-HAV-epitope response

In the peptide "sandwich" assay, wells were coated with peptide which represents the desired heterologous epitope (HAV) and then saturated with a buffer solution containing bovine serum albumin. Serum plasma of the animals and appropriate controls were incubated and the binding was detected by a second incubation with an anti-mouse IgG antibody which was enzyme-linked. The unbounded antibodies were removed by washing and the enzyme activity was measured as described above.

Table V summarizes the immune response of the immunized mice.

Table V

| Immune Response in Animals - Measurement of the Antibody-Titer | | |
|---|---|---|
| No. of imm. mice | anti-HBsAg (Units/ml) | anti-HAV (Units/ml) |
| 20 | 465 | 280 |

Example 15

Description of Vectors used for the expression of a peptide wherein the heterologous epitope is of HIV origin and/or the T-cell type is of HBV-pre-S1 origin.

Plasmid pHBl1

The plasmid pHB1 containing the H2K promoter, a pBR322 residue and the HBV-S-genes were digested with BglII and XbaI. Two molecules were expected, one 550 bp fragment (coding for pre-S and 5' terminal pat of S) was deleted and the 6.350 kb fragment of interest (containing the pBR, promoter and a fraction of the S gene) was isolated after gel electrophoresis and ligated together with a synthetic oligo nucleotide DNA sequence coding for a HIV gp160 epitope (NS 1885 - 2022) (oligo No. 131 of Table I) and a start signal upstream of the HIV fragment in the exact reading frame, with corresponding sticky ends for the ligation.

Plasmids pHBl2, pHBl3, pHBl4, pHBl5, pHBl6, pHBl7, pHBl8, pHBl9, pHBl10

The plasmids pHBl2 - pHBl10 were prepared in the same way as described above. They differs only in that way that the DNA sequences are coding for different HIV epitopes (oligonucleotide nos. 133, 135, 137, 139, 141, 143, 145, 147 and 149, respectively of Table I).

Plasmid pHBl1S1

The plasmid pHBl1 was linearized with EcoRI and ligated together with a EcoRI fragment isolated from plasmid pMT-neo K (as described above) containing the MT-promoter upstream of the neomycin resistance gene.

Plasmids pHBl2S1 - pHBl10S1

Plasmids pHBl2S1, pHBl3S1, pHBl4S1, pHBl5S1, pHBl6S1, pHBl7S1, pHBl8S1, pHBl9S1 and pHBl10S1 were prepared by the same procedure as described for plasmid pHBl1S1.

Plasmid pHBl1S2

The plasmid pHBl1 was linearized with EcoRI and ligated together with a second EcoRI fragment isolated from a plasmid pMTegpt (as described above) containing the MT promoter upstream of the egpt selection gene.

Plasmid pHBl1A

The plasmid pHBl1 was linearized with EcoRI and ligated together with a EcoRI fragment isolated from a plasmid pdhfr 3.2 containing the selection/amplification gene.

Plasmid pHT2Bl1

The plasmid pHB1 residue derived after a digestion with BglII and XabI and remvoal of the 550 bp fragment (as described above) was ligated together with a synthetic oligo-nucleotid-DNA sequence of 200 bp (oligonucleotide no. 201 of Table I) coding for the HBV-preS1-T cell epitope and one epitope of the HIV gp160 and a start signal upstream of the T cell epitope in the exact reading frame with the corresponding sticky ends. Two synthetic oligonucleotides (no.175 and no.131) were first ligated together to obtain this 200 bp oligonucleotide.

Example 16

Generation of antigen producing recipient cells by gene transfer (transfection) of desired plasmids into host cells (heterologous HIV epitope and T-cell epitope)

(1) The recipient cells were first transfected with the plasmid pHBP2S2 (containing the gene sequence of the particle forming polypeptide). A cell clone was found which expressed a high level of the monomer. A second transfection (co-transfection) was done with the plasmids pHBI1S1 and pUT1B for the identified clone. The new cell clones were analyzed by immunoassays to detect cells which were secreting the described heterologous polymeric particles.

(2) The recipient cells were transfected (cotransfected) with a mixture of plasmid pHT2BI1 together with plasmid pHBP2S1. After transfection the grown cell clones were analyzed by immunoassays to detect cell clones expressing the described heterologous polymeric particles.

(3) The same heterologous polymeric antigen will be obtained when the following plasmid combinations are used for the transfection of the recipient cells:

-pHBP2S1 + pHT1BI1

-pHBP2S1 + pHT23BI1

-pHBP2A + pHT3BI1 or

-pHBP2S2 + (pHBI1S1 + pUT2B)

-pHBP2S2 + (pHBI1S1 + pUT3B)

Plasmids pHBI2 - pHBI10 can be used as described for pHBI1.

All subsequent procedures were performed as described above.

Table VI

| Measurement of the desired epitopes after CsCl gradient | | | |
|---|---|---|---|
| Measurement by Elisa with Antibodies | | | |
| Frac. No pooled | anti-HBsAg (OD) | anti-HIV (OD) | anti-HBV-preS1 (OD) |
| 10 -12 | 1.721 | 0.581 | 0.871 |

Example 17

Immune response in animals after immunisation with the heterologous polymeric vaccine described in Example 16.

a) Detection of the anti-HBs response

Male Balb/c mice were immunised i.p. with 0.5 ml suspension containing the desired concentration of antigen. After 28 days, the mice were bled and the plasma sera were separated by centrifugation for 30 minutes at 6..00 rpm. The antibody content of the sera was measured by using an anti-HBs "sandwich" assay. The wells were coated with HBsAg (subtype adw and ayw). Serum plasma and appropriate controls were added to the wells and incubated. After washing, peroxidase conjugated HBsAg was added which bound onto the second determinant of the mouse antibody. The unbound enzyme-linked antigen was removed by washing and the enzyme activity on the solid phase was determined. The enzymatically catalyzed reaction of hydrogen peroxidase and chromogen was stopped by adding diluted sulfuric acid. The colour intensity was proportional to the anti-HBs antibody concentration of the samples and was measured by photometric comparison of the colour intensity of the unknown samples with the colour intensity of the accompanying negative and positive control sera.

b. Detection of the anti-HIV-epitope response

In the peptide "sandwich" assay, wells were coated with peptide which represents the desired heterologous epitope (HIV) and then saturated with a buffer solution containing bovine serum albumin. Serum plasma of the animals and appropriate controls were incubated and the binding was detected by a second incubation with an anti-mouse IgG antibody which was enzyme-linked. The unbounded antibodies were removed by washing and the enzyme activity was measured as described above.

c) Detection of the anti-HBV-pre-S1 T-cell epitope response

In the peptide "sandwich" assay, wells were coated with peptide which represents the desired T-cell epitope (pre-S1) and then saturated with a buffer solution containing bovine serum albumin. Serum plasma of the animals and appropriate controls were incubated and the binding was detected by a second incubation with an anti-mouse-IgG antibody which was enzyme-linked. The unbounded antibodies were removed by washing and the enzyme activity was measured as described above.

Table VII

| Immune Response in Animals - Measured Antibody Titer (Example 16(1)) | | | |
|---|---|---|---|
| No. of mice | anti-HBsAg (Units/ml) | anti-HIV ep. (Units/ml) | anti-pre-HBV-S1 T-cell (Units/ml) |
| 20 | 1.220 | 1.172 | 0.775 |

Table VIII

| Immune Response in Animals - Measured Antibody Titer (Example 16(2)) | | | |
|---|---|---|---|
| No. of mice | anti-HBsAg (Units/ml) | anti-HIV ep. (Units/ml) | anti-HBVpre-S1 T-cell (Units/ml) |
| 21 | 1.410 | 1.321 | 0.925 |

Example 18

Vectors used for the expression of a peptide wherein the heterologous epitope is of another origin

Plasmid pHBH3

The plasmid pHB1 containing the H2-promoter, a pBR322 residue and the HBsAg genes were digested with BglII and XbaI. Two molecules were generated, one 550 bp fragment was deleted and the fragment of interest (containing the H2-promoter, a pBR part and a fraction of the S-gene) was isolated after gel electrophoresis and ligated together with a synthetic oligonucleotide (oligonucleotide no. 155 of Table I) coding for a T-cell epitope of malaria and a start signal upstream of it in the exact reading frame, with corresponding sticky ends for the ligation.

Plasmid pHBH1 and pHBH2

Both plasmids were prepared in the same way as described for pHBH3. Plasmid pHBH1 carries oligonucleotide (oligonucleotide no. 155 of Table I) (coding for a malaria B-cell epitope) and plasmid pHBH2 carries oligonucleotide (oligonucleotide no. 161 of Table I) (coding for an epitope of foot and mouth disease).

Plasmid pHBH1S1, pHBH2S1, pHBH3S1

These plasmids were supplemented by adding to pHBH1, pHBH2 and pHBH3 the neomycin resistance gene as described above.

Example 19

Generation of antigen producing recipient cells by gene transfer (transfection) of desired plasmids into host cells (heterologous malaria epitope and two T-cell epitopes)

Recipient cells were transfected first (cotransfection) with the plasmids pUT1B and pHBP2S2. A cell clone was found with a stable expression of the desired monomers. A second transfection (cotransfection) was done on the identified clone with the plasmids pUT2B and pHBH3S1.

All subsequent procedures were performed as described above.

Table IX

| Measurement of the desired epitopes after CsCl gradient | | | |
|---|---|---|---|
| Measurement by Elisa with antibodies | | | |
| Frac. no. pooled | anti-HBs (OD) | anti-T-cell malaria (OD) | anti-B-cell malaria (OD) | anti-pre-S1 (OD) |
| 10 - 13 | 1.687 | 0.785 | 0.643 | 0.925 |

Example 20

Immune response in animals after immunisation with the heterologous polymeric vaccine described in Example 20.

a) Detection of the anti-HBs response and the anti-HBV-pre-S1 response was done as described in above.

22

b) Detection of the anti-Malaria (hybrid polypeptid- B-Cell epitope) response

The peptide sandwich assay wells were coated with peptide which represents the desired B-cell epitope (malaria) and then saturated with a buffer solution containing bovine serum albumin. Serum plasma of the animals and appropriate controls were incubated and the binding was detected by a second incubation with an anti-mouse-IgG antibody which was enzyme linked. The unbounded antibodies were removed by washings and the enzyme activity was measured as described above.

c) Detection of the anti-malaria-T-cell epitope response was performed as described above for the malaria B-cell epitope response, except a specific peptide representing the desired T-cell epitope (malaria) was used to coat the assay wells.

## Table X

## Immune Response in Animals - Measured Antibody Titer

| No of mice | anti HBs HBV (U/ml) | anti-T-cell malaria (U/ml) | anti B-cell malaria (U/ml) | anti-preS1 HBV (U/ml) |
|---|---|---|---|---|
| 20 | 3.005 | 1.750 | 0.877 | 2.107 |

Example 21

General Procedures

General procedures useful in practicing the present invention may be found in (1) Methods of Enzymology, volume 152, "Guide to Molecular Cloning Techniques," ed. Berger and Kimmel (Academic Press 1987), and (2) Maniatis et al., "Molecular Cloning: A Laboratory Manual," (Cold Spring Harbor Laboratory 1982), both of which are incorporated herein in their antirety by reference. Specific techniques employed are described below.

1) Digestion with Endonucleases and Isolation of Fragments

The restriction endonucleases used were:
BglII, BamHI, HindIII, EcoRI, XbaI, MstII, XhoI, Pf1MI, commercially available from Gibco/BRL with their respective restriction buffers (10x).
Unless otherwise indicated, restriction digests were performed and fragments were isolated as follows. Reactions typically contained 1-5 ug DNA.
- distilled water was added to the DNA in an eppendorf tube to a final volume of 8 ul
- 1 ul of the appropriate 10x digestion buffer was added
- 1 ul (containing 5-10 U) restriction enzyme was added and mixed carefully
- the reaction tube was incubated for 1 hour at 37° C
- digestion was stopped by adding 0.5 M EDTA (pH 8.0) to a final concentration of 10 mM

- if the DNA was analysed directly on a gel, 1 ul of gel-loading dye III (Maniatis) was added, mixed and the sample was loaded into the slots of a 0.8% agarose gel.

The agarose gel normally contains 0.8% agarose 1 x running buffer (TBE, Maniatis). Where a fragment (about 100-1000bp) was isolated from an agarose gel the agarose was increased to 1.2 to 1.4%.

2) Competent Bacterial Cells

From a dense overnight culture, 1 ml of the bacterial cell suspension was added to 100 ml fresh growth medium (L-broth). The cells were grown at 37°C to a density of $OD_{600}$ = 0.7 which was reached within 2 hours with vigorous shaking in a 500 ml Erlenmeyer flask. Growth was stopped by chilling the culture on ice for 10 minutes. From this culture, 3 ml were taken for harvesting the exponential bacterial cells at 3,000 rpm for 5 minutes. The cells were resuspended in 1.5 ml of 50 mM $CaCl_2$ in 10 mM Tris, pH 8.0, and incubated on ice for another 15 minutes. The cells were harvested once more by centrifugation at 3,000 rpm for 5 minutes and resuspended in 200 ul of 50 mM $CaCl_2$ in 10 mM Tris, pH 8.0, and used directly.

3) Transformation of Competent Bacterial Cells

The DNA to be transformed was suspended in 10 mM Tris, pH 7.5, 1 mM EDT 70 ul and added to the 200 ul bacterial cell suspension for DNA take-up. The mixture was incubated on ice for 30 minutes and then 1 ml L-broth was added. The mixture was incubated at 42°C for 2 minutes and at 37°C for 40 minutes.

After the incubation, the cells were spread on agar plates containing 50 ug ampicillin/ml agar at volumes of 50-300 ul cell suspension per plate. The agar plates were incubated at 37°C overnight. After this incubation period, single isolated bacterial colonies were formed.

4) Plasmid DNA Isolation

1 liter of plasmid-bearing cells was grown to 0.5 $OD_{600}$ in L-broth and amplified for 20 hours with 200 ug/ml chloramphenicol. The culture was then centrifuged at 4,000 rpm for 20 minutes in JA-10 rotor, 4°C. The pellet was resuspended in 18 ml cold 25% sucrose, 50 mM Tris, pH 8.0, transferred to a 250 ml Erlenmeyer flask and kept on ice. 6 ml 5mg/ml lysozyme in 250 mM Tris, pH 8.0 was added and the mixture was left to stand 10-15 minutes. 6 ml 250 mM EDTA, pH 8.0, was added, mixed gently and incubated for 15 minutes on ice. 30 ml detergent (0.01% Triton X-100: 60 mM EDTA, pH 8.0: 50 mM Tris, pH. 8.0) was added and the mixture was incubated for 30 minutes on ice. After incubation, the mixture was centrifuged at 25,000 rpm 90 minutes in SW28 rotor, 4°C.

Pronase was added to supernatant fluid to 250 ug/ml and incubated 30 minutes, 37°C. The solution was extracted with phenol once with 1.2 volume phenol equilibrated with 10 mM Tris, pH 8.0, 1 mM EDTA. The aqueous layer was removed. Sodium acetate was then added to a final concentration of 300 mM, followed by the addition of 3 volumes cold 100% ethanol and thorough mixing. The mixture was stored at -20°C overnight.

The mixture was thawed and centrifuged. The pellet was resuspended in 6 ml 10 mM Tris, 10 mM EDTA, pH 8.0. 9.4 g CsCl and 0.65 ml of 6 mg/ml ethidium bromide were added and the volume was brought up to 10 ml with sterile double-distilled water. The 10 ml aliqnots were put into Beckman heat-sealable gradient tubes and centrifuged, 50,000 rpm, 48 hours in Ti70.1 Beckman rotor.

Plasmid bands were visualized with UV and removed with syringe and 18 gauge needle by piercing the side of the tube. Ethidium bromide was removed from the plasmid fractions by 3 successive extractions with equal volumes of isobutanol. Fractions were then (1) dialyzed against one 2-liter lot of 10 mM Tris, pH 7.4, 1 mM EDTA, pH 7.5, 5 mM NaCl for 2 hours or more at 4°C; and (2) phenol extracted once with 1 3 volume phenol equilibrated as above. Sodium acetate was then added to a final concentration of 300 mM, followed by addition of two volumes of 100% ethanol. Precipitate formed at -20°C overnight, or at -70°C for 30 minutes.

### 5) Mini-Plasmid Preparation

1 ml of an overnight bacteria culture was put into an eppendorf tube and centrifugated for 20 minutes. The supernatant was removed. 100 ul of 50 mM glucose, 25 mM Tris (pH 8.0), 10 mM EDTA (pH 8.0) was added to the pellet, mixed by vortex and incubated for 5 minutes at room temperature. 200 ul of 0.2 N NaOH, 1% SDS was added, mixed by vortex and incubated for 5 minutes on ice. 150 ul 3 M Sodium acetate (pH 4.8) was added, mixed by vortex and incubated for 5 minutes on ice. After centrifugation for 5 minutes at 13,000 rpm the supernatant was decanted into a fresh eppendorf tube. 3 volumes of 100% ethanol were supplemented, mixed well ad incubated for 30 minutes at -80°C, then centrifuged for 10 minutes at 13,000 rpm. The ethanol was removed, the pellet washed with 70% ethanol, lyophilized and dissolved in 20 ul distilled water. 5 ul of this plasmid DNA solution were used directly for restriction analysis.

### 6) Nick Translation

Nick translation was performed according to Rigby et al., J. Mol. Biol., Vol. 113, pp. 237-251, 1977, which is incorporated herein by reference. The reaction mixture for $^{32}$P-labeling of DNA contained 0.5 ug of a HBV fragment, in a total volume of 30 ul with 50 mM Tris, pH 7.8 5 mM $MgCl_2$, 10 mM mercaptoethanol, 0.1 mM dATP, 0.1 mM dGTP, 0.1 mM dTTP, 50 uCi $^{32}$P-dCTP, 10 units DNA polymerase I; 3 ul of a 2 x $10^{-5}$ fold dilution of 1 mg/ml DNase I and is incubated for 90 minutes at 15°C, yielding $3 \times 10^6$ to $12 \times 10^6$ total cpm, i.e. $1 \times 10^7$ to $5 \times 10^7$ cpm/ug DNA.

### 7) Southern Blot Analysis

To characterize the organization within the host cell genome of the vectors of this invention, chromosomal DNA from cell lines producing particles of this invention were isolated and digested with the appropriate restriction enzyme(s) and analysed by the method of Southern (J. Mol. Biol., Vol. 98, pp. 503-517, 1975), which is incorporated herein by reference, using a $^{32}$P-labeled DNA probe. Following digestion of the chromosomal DNA (20 ug) with the restriction enzyme BglII, the resulting fragments were separated by 0.7% agarose gel electrophoresis. Thereafter, the DNA was denatured by exposing to 366 nm UV light for 10 minutes and by incubation in a solution of 0.5 M NaOH and 1 M NaCl for 45 minutes. The gels were neutralized by incubation in 0.5 M Tris, 1.5 M NaCl, pH 7.5 for 60 minutes. The DNA was transferred to a nitrocellulose filter by soaking in 3 M NaCl, 0.3 M Sodiumcitrate (20 x SSC) for 20 hours through the gel by covering the top of the nitrocellulose filter with a staple of dry paper towels. The nitrocellulose filter was kept for 2 hours in a vacuum oven at 80 C. A radioactive DNA probe from the BglII fragment of the pHBV (2.3 kb) was prepared by nick translation.

For hybridization with the DNA probe, the nitrocellulose filter was sealed in a plastic bag containing 10 ml of prehybridization mixture: 50% formamide, 5 x SSC, 50 mM Sodiumphosphate, pH 7.0, 5 x Denhardt's solution, 250 ug/ml denatured salmon sperm DNA. The filter was incubated in this mixture for 4 hours at 45°C, after which the pre-hybridization mixture was replaced by the hybridization mixture: 50% formamide, 5 x SSC, 20 mM Sodiumphosphate, pH 7.0, 1 x Denhardt's solution. 100 ug/ml denatured salmon sperm DNA, 5 x $10^5$ cmp/ml $^{32}$P-probe. The filter, after incubating in the hybridization mix for 18 hours at 45°C, was washed three times, 5 minutes each, in 0.1 x SSC, 0.1% SDS at 50°C. The filter was dried at 60°C for 10 minutes and exposed to two X-ray films (XAR-5, KODAK) between two intensifying screens and kept at -80°C. The first X-ray film is developed after 3 days' exposure; the second film after 7 days' exposure.

### 8) Preparation of Mammalian Cells and DNA Precipitate for Transfection

The recipient cells ($C_{127}$, L- or CHO-cells (available from ATCC) were seeded in normal growth medium (DNEM + 5% Fetal Calf Serum, Glycose and Glutamin) into petri-dishes (1-2 x $10^6$ cells per dish, ⌀ 10 cm) at day 1. The next day the medium was removed (4 hours before the DNA precipitate was added onto the cells), and the cells were washed twice with 1 x PBS. Then 8 ml DMEM (complemented with Hepes - final concentration 10 mM) without FCS were added. 4 hours later the DNA precipitate (prepared as described below) was added to the cells. Again after 4 hours the medium was removed, 3 ml of Glycerol-Mix (50 ml 2 x HBS buffer, 30 ml glycerol, 120 ml distilled water) were added. The Glycerol-Mix was immediately removed after an incubation at 37° C for 3 minutes and the cells were washed with 1 x PBS. The cells were cultivated overnight with 8 ml of DMEM with 10% FCS.

The following day the cells were recovered from the dish by treating with Trypsin-EDTA-Solution (0.025% Trypsin + 1 mM EDTA). Afterwards, to remove the Trypsin-EDTA the cells were washed with 1 x PBS, suspended in DMEM with 10% FCS and distributed into 24 costar-well-plates (cells from one dish into one 24-well-plate). When the cells had grown well, selection medium was added (concentration 0.5 - 1 mg ml. of neomycin or a medium comprising $C_{27}$, L- or CHO-cells (available from ATCC) (egpt.) for example). The medium was changed every week. The first growing cell colonies were seen after 2 weeks.

To 10 ug of plasmid DNA and 20 ug of carrier-DNA (salmon-sperm DNA, calf-thymus DNA) TE-buffer (10 mM Trix-HCl, 1 mM EDTA, pH 7.05) was added to a final volume of 440 ul and mixed together with 60 ul 2 M $CaCl_2$. Then the same amount of 2x HBS (Hepes 50 mM, NaCl 280 mM, $Na_2HPO$ 1.5 mM, pH 7.05) was added and mixed well. The precipitation solution was incubated for 30 minutes at 37° C and added directly to the cells which should be transfected.

From the foregoing, it will be obvious to those skilled in the art that various modifications in the above-described compositions and methods can be made without departing from the spirit and scope of the invention. Accordingly, the invention may be embodied in other specific forms without departing from the spirit or essential characteristics thereof. Present embodiments, therefore, are to be considered in all respects as illustrative and not restrictive, the scope of the invention being indicated by the appended claims rather than be the foregoing description, and all changes which come within the meaning and range of equivalency of the claims are therefor intended to be embraced therein.

## Claims

1. An immunogenic particle comprising a plurality of first peptide monomers and a plurality of second peptide monomers,
each of said first peptide monomers comprising:
an amino acid sequence corresponding to a substantial portion of a peptide which upon secretion will form particles which are at least 10 nm in diameter, and
an amino acid sequence corresponding to a heterologous epitope,
each of said second peptide monomers comprising an amino acid sequence corresponding to a substantial portion of a peptide which upon secretion will form particles which are at least 10 nm in diameter, each of said second peptide monomers being free of an amino acid sequence corresponding to a heterologous epitope, wherein said first peptide monomer and said second peptide monomer are not identical but are capable of binding together.

2. An immunogenic particle comprising a plurality of first peptide monomers, a plurality of second peptide monomers and a plurality of third peptide monomers,
each of said first peptide monomers comprising:
an amino acid sequence corresponding to a substantial portion of a peptide which upon secretion will form particles which are at least 10 nm in diameter, and
an amino acid sequence corresponding to a heterologous epitope.
each of said second peptide monomers comprising:
an amino acid sequence corresponding to a substantial portion of a peptide which upon secretion will form particles which are at least 10 nm in diameter,
each of said second peptide monomers being free of an amino acid sequence corresponding to a heterologous epitope, and
each of said third peptide monomers comprising:
an amino acid sequence corresponding to a substantial portion of a peptide which upon secretion will form particles which are at least 10 nm in diameter, and
an amino acid sequence corresponding to a T-cell epitope.
wherein said first peptide monomers, said second peptide monomers and said third peptide monomers are capable of binding to one another.

3. An immunogenic particle comprising a plurality of first peptide monomers and a plurality of second peptide monomers,
each of said first peptide monomers comprising:
an amino acid sequence corresponding to a substantial portion of a peptide which upon secretion will form particle which are at least 10 nm in diameter,
an amino acid sequence corresponding to a heterologous epitope, and

an amino acid sequence corresponding to a T-cell epitope,

each of said second peptide monomers comprising:

an amino acid sequence corresponding to a substantial portion of a peptide which upon secretion will form particles which are at least 10 nm in diameter,

each of said second peptide monomers being free of an amino acid sequence corresponding to a heterologous epitope,

wherein said first peptide monomers and said second peptide monomers are capable of binding together.

4. An immunogenic particle of claim 1, 2 or 3 wherein said peptide which upon secretion will form particles which are at least 10 nm in diameter is a peptide selected from the group consisting of HBV S peptide, HBV core antigen, polio surface antigen, Hepatitis A surface antigen, Hepatitis A core antigen, HIV surface antigen and HIV core antigen.

5. An immunogenic particle comprising a plurality of first peptide monomers and a plurality of second peptide monomers,

each of said first peptide monomers comprising:

an amino acid sequence corresponding to a substantial portion of the HBV S peptide, and

an amino acid sequence corresponding to a heterologous epitope,

each of said second peptide monomers comprising:

an amino acid sequence corresponding to a substantial portion of the HBV S peptide,

each of said second peptide monomers being free of an amino acid sequence corresponding to a heterologous epitope,

wherein said first peptide monomer and said second peptide monomer are not identical but are capable of binding together.

6. An immunogenic particle comprising a plurality of first peptide monomers, a plurality of second peptide monomers, and a plurality of third peptide monomers,

each of said first peptide monomers comprising:

an amino acid sequence corresponding to a substantial portion of the HBV S peptide. and

and amino acid sequence corresponding to a heterologous epitope,

each of said second peptide monomers comprising:

an amino acid sequence corresponding to a substantial portion of the HBV S peptide,

each of said second peptide monomers being free of an amino acid sequence corresponding to a heterologous epitope, and

each of said third peptide monomers comprising:

an amino acid sequence corresponding to a substantial portion of the HBV S peptide,

an amino acid sequence corresponding to a T-cell epitope,

wherein said first peptide monomers, said second peptide monomers and said third peptide monomers are capable of binding together.

7. An immunogenic particle comprising a plurality of first peptide monomers and a plurality of second peptide monomers,

each of said first peptide monomers comprising:

an amino acid sequence corresponding to a substantial portion of the HBV S peptide,

an amino acid sequence corresponding to a heterologous epitope, and

an amino acid sequence corresponding to a T-cell epitope,

each of said second peptide monomers comprising an amino acid sequence corresponding to a substantial portion of the HBV S peptide.

wherein said first peptide monomers and said second peptide monomers are capable of binding together.

8. An immunogenic particle of claim 5, 6 or 7 wherein each of said first peptide monomers comprises an amino acid sequence corresponding to an HBV S peptide epitope.

9. An immunogenic particle of claim 5, 6 or 7 wherein each of said second peptide monomers comprises an amino acid sequence corresponding to all of the HBV S peptide.

10. An immunogenic particle of claim 5, 6, 7 wherein said heterologous epitope is a viral epitope.

11. An immunogenic particle of claim 10 wherein said viral epitope is selected from the group consisting of a Hepatitis A epitope, a non-A Hepatitis epitope, a non-B Hepatitis epitope, an HIV epitope, a polio epitope and a foot and mouth disease virus epitope.

12. An immunogenic particle of claim 5, 6 or 7 wherein said heterologous epitope is a parasitic epitope.

13. An immunogenic particle of claim 12 wherein said parasitic epitope is a malaria parasite epitope.

14. An immunogenic particle of claim 5, 6 or 7 wherein said heterologous epitope has an amino acid sequence encoded by one of the nucleotide sequences described in Table I.

15. An immunogenic particle of claim 5, 6 or 7 wherein said T-cell epitope has an amino acid sequence encoded by one of the nucleotide sequences described in Table I.

16. A recombinant DNA molecule comprising a monomer coding region, said monomer coding region comprising a first DNA sequence and a second DNA sequence,

said first DNA sequence encoding for expression of an amino acid sequence corresponding to a substantial portion but not all of the HBV S peptide,

said second DNA sequence encoding for expression of an amino acid sequence corresponding to a heterologous epitope,

said first DNA sequence and second DNA sequence (1) being in the same reading frame, (2) encoding for respective discrete regions of a single peptide, and (3) being operatively linked to an expression control sequence in said recombinant DNA molecule located outside of said monomer coding region.

17. A recombinant DNA molecule of claim 16 wherein said monomer coding region consists essentially of said first DNA sequence and said second DNA sequence.

18. A recombinant DNA molecule comprising a first DNA sequence and a second DNA sequence,

said first DNA sequence encoding for expression of an amino acid sequence corresponding to a substantial portion of the HBV S peptide,

said second DNA sequence encoding for expression of an amino acid sequence corresponding to a T-cell epitope,

said first DNA sequence and second DNA sequence (1) being in the same reading frame, (2) encoding for respective discrete regions of a single peptide, and (3) being operatively linked to an expression control sequence in said recombinant DNA molecule.

19. A recombinant DNA molecule comprising a first DNA sequence, a second DNA sequence and a third DNA sequence,

said first DNA sequence encoding for expression of an amino acid sequence corresponding to a substantial portion of the HBV S peptide,

said second DNA sequence encoding for expression of an amino acid sequence corresponding to a heterologous epitope,

said third DNA sequence encoding for expression of an amino acid sequence corresponding to a T-cell epitope,

said first DNA sequence, second DNA sequence and third DNA sequence (1) being in the same reading frame, (2) encoding for respective discrete regions of a single peptide, and (3) being operatively linked to an expression control sequence in said recombinant DNA molecule.

20. A host cell transfected with the recombinant DNA molecule of claim 16, 17, 18, or 19.

21. A host cell cotransfected with a first recombinant DNA molecule and a second recombinant DNA molecule, said first recombinant DNA molecule being a recombinant DNA molecule of claim 16, 17, 18, or 19, and said second recombinant DNA molecule comprising a DNA sequence encoding for expression of an amino acid sequence corresponding to a substantial portion of the amino acid sequence of the HBV S peptide.

22. A host cell of claim 21 wherein said second recombinant DNA molecule comprises a DNA sequence encoding for expression of an amino acid sequence corresponding to all of the amino acid sequence of the HBV S peptide.

23. A peptide comprising a first discrete region and a second discrete region,

said first region corresponding to the amino acid sequence of a heterologous epitope,

said second region corresponding to a substantial portion of but not all of the HBV S peptide.

24. A peptide of claim 23 wherein said peptide consists essentially of said first discrete region and said second discrete region.

25. A peptide comprising a first discrete region and a second discrete region,

said first region corresponding to the amino acid sequence of a T-cell epitope,

said second region corresponding to a substantial portion of the HBV S peptide.

26. A peptide comprising a first discrete region, a second discrete region and a third discrete region,

said first region corresponding to the amino acid sequence of a heterologous epitope,

said second region corresponding to the amino acid sequence of a T-cell epitope,

said third region corresponding to a substantial portion of the HBV S peptide.

27. A pharmaceutical preparation comprising:

immunogenic particles of claim 1, 2, 3, 5, 6, 7 or 11 in sufficient concentration to elicit an immune response upon administration of said preparation, and

a suitable carrier.

28. A preparation useful for production of antibodies, said preparation comprising:

immunogenic particles of claim 1, 2, 3, 5, 6, 7 or 11 in sufficient concentration to cause production of antibodies upon administration of said preparation to an individual, and

a suitable carrier.

29. A preparation useful for production of antibodies, said preparation comprising:

peptides of claim 23, 24, 25 or 26 in sufficient concentration to cause production of antibodies upon administration of said preparation to an individual, and

a suitable carrier.

30. A process for producing a transfected host cell, said process comprising:

provided host cells which have been made competent for uptake of DNA,

exposing said host cells to a first preparation of DNA comprising the recombinant DNA molecule of claim 16, 17 18 or 19,

allowing under suitable conditions said host cells to take up DNA from said first preparation of DNA, and

selecting for host cells which have taken up exogenous DNA.

31. The process for producing a transfected host cell of claim 30, said process further comprising, prior to exposing said host cells to said first DNA preparation, exposing said host cells to a second preparation of DNA comprising a DNA molecule encoding for a peptide including the amino acid sequence of the HBV S peptide and allowing under suitable conditions said host cells to take up DNA from the second preparation of DNA.

32. The process for producing a transfected host cell of claim 30, said process further comprising, after exposing said host cells to said first DNA preparation, exposing said host cells to a second preparation of DNA comprising a DNA molecule encoding for a peptide including the amino acid sequence of the HBV S peptide and allowing under suitable conditions said host cell to take up DNA from said second preparation of DNA.

33. The process for producing a transfected host cell of claim 30, said first DNA preparation further comprising a DNA molecule encoding for a peptide including the amino acid sequence of the HBV S peptide.

34. A method of producing a peptide, said method comprising:

preparing a recombinant DNA molecule of claim 16, 17, 18 or 19,

transfecting a host cell with said recombinant DNA molecule

culturing said host cell under conditions allowing expression and secretion of protein by said host cell, and

collecting the peptide produced as a result of expression of DNA sequence within said recombinant DNA molecule.

35. The method of producing a peptide of claim 34 wherein said peptide produced as a result of the expression of DNA sequences within said recombinant DNA molecule contains an amino acid sequence corresponding to less than the entire coding region of said recombinant DNA molecule.

36. A method of producing immunogenic particles, said method comprising:

preparing a recombinant DNA molecule of claim 16, 17, 18 or 19,

transfecting a host cell with said recombinant DNA molecule,

culturing said host cell under conditions allowing expression and secretion of protein by said host cell, and

allowing under suitable conditions the aggregation of peptide monomers produced as a result of expression of exogenous DNA sequences within said host cell.

37. The method of producing immunogenic particles of claim 36, said method further comprising, prior to transfecting said host cell with said recombinant DNA molecule, transfecting said host cell with a DNA molecule encoding for a peptide including the amino acid sequence of the HBV S peptide.

38. The method of producing immunogenic particles of claim 36, said method further comprising, after transfecting said host cell with said recombinant DNA molecule, transfecting said host cell with a DNA molecule encoding for a peptide including the amino acid sequence of the HBV S peptide.

39. The method of producing immunogenic particles of claim 36, said method further comprising, simultaneously transfecting said host cell with said recombinant DNA molecule and a DNA molecule encoding for a peptide including the amino acid sequence of the HBV S peptide.

40. A method of manufacturing a pharmaceutical preparation, said method comprising:

preparing a recombinant DNA molecules of claim 16, 17, 18 or 19,

transfecting a host cell with said recombinant DNA molecule,

culturing said host cell under conditions allowing expression and secretion of protein by said host cell,

29

allowing under suitable conditions the aggregation of peptides produced as a result of expression of DNA sequences within said host cell to form immunogenic particles, and

combining said immunogenic particles with a suitable carrier such that said immunogenic particles are present in sufficient concentration to cause production of antibodies upon administration of said preparation to an individual.

41. The method of manufacturing a pharmaceutical preparation of claim 40, said method further comprising, prior to transfecting said host cell with said recombinant DNA molecule, transfecting said host cell with a DNA molecule encoding for a peptide including the amino acid sequence of the HBV S peptide.

42. The method of manufacturing a pharmaceutical preparation of claim 40, said method further comprising, after transfecting said host cell with said recombinant DNA molecule, transfecting said host cell with a DNA molecule encoding for a peptide including the amino acid sequence of the HBV S peptide.

43. The method of manufacturing a pharmaceutical preparation of claim 40, said method further comprising, simultaneously transfecting said host cell with said recombinant DNA molecule and a DNA molecule encoding for a peptide including the amino acid sequence of the HBV S peptide.

44. A method of manufacturing a preparation useful for production of antibodies, said method comprising:

preparing a recombinant DNA molecule of claim 16, 17, 18 or 19,

transfecting a host cell with said recombinant DNA molecule,

culturing said host cell under conditions allowing expression and secretion of protein by said host cell,

allowing under suitable conditions the aggregation of peptides produced as a result of expression of DNA sequences within said host cell to form immunogenic particles, and

combining said immunogenic particles with a suitable carrier such that said immunogenic particles are present in sufficient concentration to cause production of antibodies upon administration of said preparation to an individual.

45. The method of manufacturing a preparation useful for production of antibodies of claim 44, said method further comprising, prior to transfecting said host cell with said recombinant DNA molecule, transfecting said host cell with a DNA molecule encoding for a peptide including the amino acid sequence of the HBV S peptide.

46. The method of manufacturing a preparation useful for production of antibodies of claim 44, said method further comprising, after transfecting said host cell with said recombinant DNA molecule, transfecting said host cell with a DNA molecule encoding for a peptide including the amino acid sequence of the HBV S peptide.

47. The method of manufacturing a preparation useful for production of antibodies of claim 44, said method further comprising, simultaneously transfecting said host cell with said recombinant DNA molecule and a DNA molecule encoding for a peptide including the amino acid sequence of the HBV S peptide.